# EUROPEAN PATENT SPECIFICATION

(11) **EP 2 209 521 B1**
(45) Date of publication and mention of the grant of the patent: **02.10.2013**
(21) Application number: 07835205.1
(22) Date of filing: 14.11.2007
(51) Int. Cl.: A61N 1/05

(54) **A METHOD OF PRODUCING A RING ELECTRODE OF AN IMPLANTABLE LEAD**
VERFAHREN ZUR HERSTELLUNG EINER RINGELEKTRODE EINER IMPLANTIERBAREN LEITUNG
PROCÉDÉ DE PRODUCTION D'UNE ÉLECTRODE ANNULAIRE POUR SONDE IMPLANTABLE

(43) Date of publication of application: 28.07.2010
(73) Proprietor: St. Jude Medical AB, 175 84 Järfälla (SE)
(72) Inventor: HILL, Rolf, S-175 55 Järfälla (SE); ÖRNBERG, Andreas, S-175 79 Järfälla (SE); MICSKI, Eva, S-141 43 Huddinge (SE); DJURLING, Henrik, S-175 65 Järfälla (SE); NYGREN, Mats, S-167 73 Bromma (SE)
(74) Representative: Sjölander, Henrik
(86) International application number: PCT/SE2007/001005
(87) International publication number: WO 2009/064221

(56) References cited:
- EP-A1- 1 162 022
- EP-A1- 1 162 022
- EP-A2- 0 709 111
- EP-A2- 0 709 111
- WO-A2-2006/020090
- WO-A2-2006/020090
- WO-A2-2006/029174
- US-A- 4 590 950
- US-A- 4 590 950
- US-A- 5 685 306
- US-B1- 6 384 365
- US-B1- 6 384 365
- YU L-G ET AL: "Effect of spark plasma sintering on the microstructure and in vitro behavior of plasma sprayed HA coatings", BIOMATERIALS, ELSEVIER SCIENCE PUBLISHERS BV., BARKING, GB, vol. 24, no. 16, 1 July 2003 (2003-07-01), pages 2695-2705, XP004421035, ISSN: 0142-9612, DOI: DOI:10.1016/S0142-9612(03)00082-6

## Description

### TECHNICAL FIELD

The present disclosure generally relates to implantable leads, and in particular to ring electrodes for usage in such implantable leads.

### BACKGROUND

Body implantable electrical leads form the electrical connection between an implantable medical device (IMD), such as cardiac pacemaker, cardiac defibrillator or cardioverters, and body tissue, such as the heart, which is to be electrically stimulated. As is well known, the leads connecting the IMD with the tissue may be used for pacing/ defibrillation and for sensing electrical signals produced by the tissue.

The implantable leads of today are complex arrangements, generally including multiple different lead elements of different materials and therefore having different characteristics. This makes the assembly process of a lead time consuming and complex. Furthermore, in most implantable leads at least some of the lead elements are connected through welding. Though this may work satisfactory for elements of same materials, welding elements having different material characteristics may impose new problems. There is risk that a weld between different materials may become brittle. Thus, there is generally a need of connecting lead elements of different materials and different material characteristics.

EP 0 709 111 discloses a medical electrical lead system having a torque transfer stylet with an elongated stylet wire. The proximal and distal sections of the stylet wires have a first diameter, whereas an intermediate section has a second, smaller diameter. The intermediate section has a length of torque coil wound in a coiled diameter which is the same as the first diameter.

### SUMMARY

There is therefore a need for a lead manufacturing process that allows connecting lead elements of different materials. There is also a need for implantable electrical leads, where a risk of forming brittle welds is reduced. The present invention overcomes these and other drawbacks of the prior art arrangements.

It is a general object of the present invention to provide a ring electrode of an implantable medical lead having interconnected electrode material of differing materials.

It is another object of the invention to provide a manufacture of ring electrodes involving spark plasma sintering for directly or indirectly connecting an electrode member to a coil adapter.

These and other objects are met by the invention as defined by the accompanying patent claims.

Briefly, the present invention involves an implantable medical, electrical lead connectable to an implantable medical lead. The lead comprises a ring electrode in connection with its distal end. The ring electrode comprises an electrode member made of a first conducting material, such as titanium. This electrode member constitutes the portion of the ring electrode that provides stimulating pulses to adjacent tissue and/or sense the electrical activity of the tissue following implantation.

The ring electrode also comprises a coil adaptor made of a second conducting material, such as a metal alloy, e.g. a nickel-cobalt-chromium-molybdenum alloy. This adaptor has a proximal end connectable to a lead conductor that electrically connects the coil adaptor to a terminal electrode at the proximal end of the lead.

The coil adaptor is mechanically and electrically connected, directly or indirectly, to the electrode member at least partly by spark plasma sintering. This metal-connecting technique allows elements of different material characteristics to be reliably inter-connected even though the two materials cannot be securely inter-connected through traditional welding.

In a particular embodiment, the ring electrode also comprises a weld adapter functioning as a bridging unit between the electrode member and the coil adapter. In such a case, the weld adapter is electrically and mechanically connected to at least one of the electrode member and the coil adaptor through spark plasma sintering. The other element connection can be made with tradition welding as long as the two elements (electrode member and weld adaptor or coil adaptor and weld adaptor) are made or the same or at least inter-weldable materials.

The invention also teaches a method of producing a ring electrode of an implantable medical lead.

The invention offers the following advantages:
- Allows reliable electrical and mechanical inter-connection of ring electrode elements of different materials; and
- Simplifies the assembly process.

Other advantages offered by the present invention will be appreciated upon reading of the below description of the embodiments of the invention.

### SHORT DESCRIPTION OF THE DRAWINGS

The invention together with further objects and advantages thereof, may best be understood by making reference to the following description taken together with the accompanying drawings, in which:
Fig. 1 is a side view of an implantable lead according to an embodiment of the present invention;
Fig. 2 is a schematic overview of a subject having an implantable medical device connected to an implantable lead according to an embodiment of the present invention;
Fig. 3 is an axial cross section view of the distal portion of an implantable medical lead according to an embodiment of the present invention;
Fig. 4A is a side view of an electrode member according to an embodiment of the present invention;
Fig. 4B is a side view of a coil adapter according to an embodiment of the present invention;
Fig. 4C is a side view of a ring electrode formed by the electrode member and coil adapter of Figs. 4A and 4B;
Fig. 5A is a side view of a weld adapter according to an embodiment of the present invention;
Fig. 5B is a side view of a coil adapter according to another embodiment of the present invention;
Fig. 5C is a side view of ring electrode member formed by the weld adapter and coil adapter of Figs. 5A and 5B;
Fig. 5D is a side view of an electrode member according to an embodiment of the present invention;
Fig. 5E is a side view of a ring electrode formed by the electrode member and ring electrode member of Figs. 5C and 5D;
Fig. 6A is a side view of a coil adapter according to a further embodiment of the present invention;
Fig. 6B is a side view of ring electrode member formed by the coil adapter of Fig. 6A and a weld adapter;
Fig. 6C is a side view of a ring electrode formed by the electrode member and ring electrode member of Figs. 5D and 5E;
Fig. 7 is an axial cross section view of the distal portion of an implantable medical lead according to another embodiment of the present invention;
Fig. 8 is a flow diagram illustrating a method of producing a ring electrode according to an embodiment of the present invention;
Fig. 9 is a flow diagram illustrating two of the production steps of the method in Fig. 8 according to an embodiment of the present invention;
Fig. 10 is a flow diagram illustrating two of the production steps of the method in Fig. 8 according to another embodiment of the present invention;
Fig. 11 is a schematic block diagram of a spark plasma sintering apparatus that can be used according to the present invention; and
Figs. 12A and 12B are scanning electron microscope images of a Ti-MP35N^{®} connection at 500 x magnification (Fig. 12A) and 4000 x magnification (Fig. 12B).

### DETAILED DESCRIPTION

Throughout the drawings, the same reference characters will be used for corresponding or similar elements.

The present invention relates to implantable medical leads or catheters, and in particular to a distal end arrangement thereof and such distal lead arrangements having a ring electrode. The leads of the invention are adapted for connection to different implantable medical devices (IMDs), such as pacemakers, cardioverters, defibrillators and other implantable electrical medical devices.

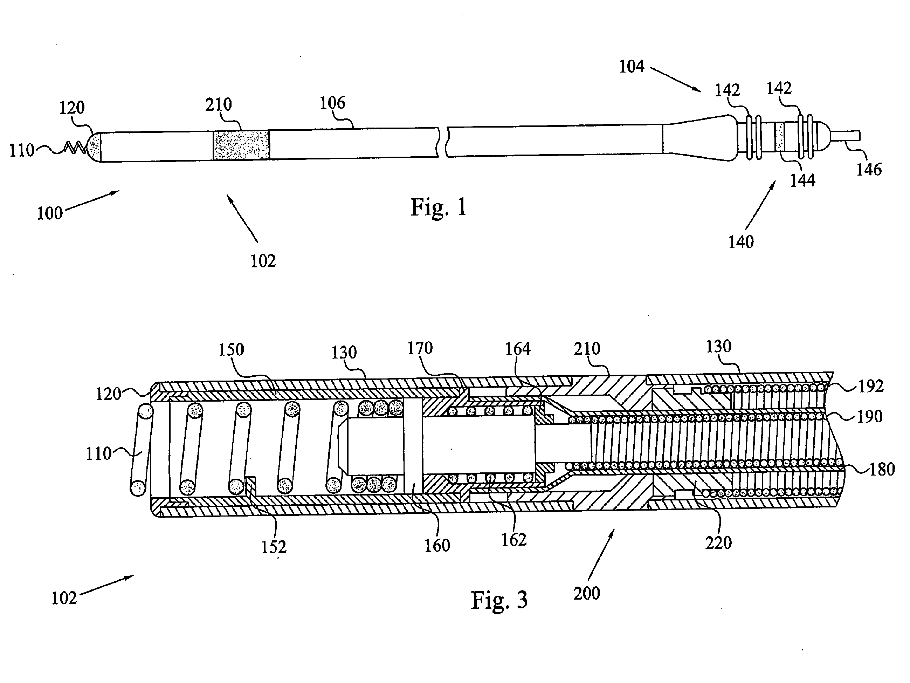

Fig. 1 is a schematic illustration of an implantable lead 100 according to an embodiment of the present invention. The lead 100 comprises a lead body 106 extending a long a central, longitudinal axis. The lead 100 has a proximal end 104 carrying a connector assembly 140 for electrically connecting the lead body 106 to an IMD. The lead 100 also has a distal end 102 comprising a header with electrodes 210 and fixation elements 110. The lead 100 has non-limitedly been illustrated in the form of a so-called active fixation medical lead 100, implying that the fixation element 110 is in the form of a helical, screw-in fixation element 110 adapted to be extended so as to project from the distal end of the header. The helical screw-in fixation element 110 is preferably active electrically so as to function as an electrode when implanted to stimulate selected tissue, such as cardiac tissue, and/ or sense electrical activity of the tissue. Consistent with teachings well known in the art, one or more portions of such a helical electrode 110 may be electrically insulated along its length. The helical electrode 110 not only has a stimulating and/or sensing function but also serves to anchor or stabilize the distal lead portion 102 relative to the tissue.

The distal lead portion 102 also comprises a ring electrode 200 or indifferent electrode according to the present invention. This ring electrode 200 is provided for electrically stimulating adjacent tissue and/or for sensing electrical activity of tissue.

The connector assembly 140 at the proximal lead end 104 is adapted to electrically and mechanically couple the lead body 106 to the IMD. The assembly 140 comprises terminal contacts in the form of a tubular, rotatable pin terminal contact 146, often denoted connector pin 146, and a ring terminal contact 144, generally referred to as connector ring 144. These two contacts 144, 146 are positioned to engage corresponding electrical terminals within a receptacle in the IMD. In order to prevent ingress of body fluid into the IMD receptacle, the connector assembly 140 may be provided with spaced-apart sets of seals 142, well known in the art.

The present invention is not limited to be implemented into active fixation medical leads having electrically conducting helical screw-in fixation elements. In clear contrast, the teachings of the invention can be applied to leads having helical screw-in fixation elements that are not employed as stimulating/sensing electrodes, i.e. being made of non-conducting material and/or having no electrical contact with electrical terminals in the IMD. The invention can also be applied to passive fixation leads, where the helical screw-in fixation element is replaced by, for instance, a collar, tines or fines for anchoring the lead body to a selected tissue.

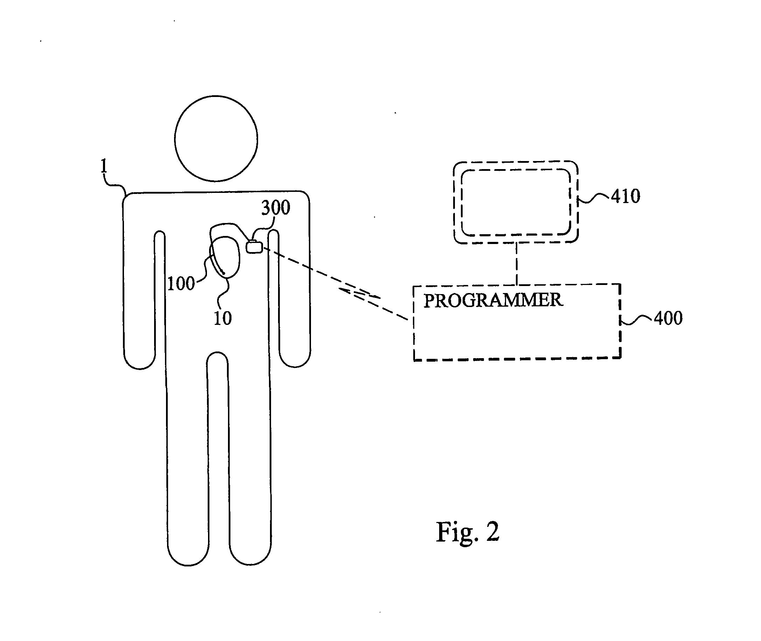

Fig. 2 is a schematic overview of a subject 1 equipped with an IMD 300 connected to the subject's heart 10. The IMD 300 is illustrated as a device that monitors and/or provides therapy to the heart 10 of the patient 1, such as a pacemaker, defibrillator or cardioverter. However, the present invention is not limited to cardiac-associated IMDs but may also be practiced with other implantable medical devices, such as drug pumps, neurological stimulators, physical signal recorders, oxygen sensors, or the like, as long as the IMD 300 is equipped with or is connected to at least one medical lead 100 according to the present invention.

The IMD 300 can wirelessly communicate with an external device 400, non-limitedly illustrated as a programmer 400 in the figure. The external device 400 could alternatively be a physician's workstation, a home monitoring device or actually any data processing unit having capability of receiving data collected by the IMD 300 and preferably sending instructions and commands to the IMD 300. The external device 400 is preferably connected to a display screen 410 allowing display of the collected diagnostic parameters and data

Fig. 3 is a cross-sectional view of the distal end 102 of the implantable lead of Fig. 1. The helix electrode 110 is mechanically and electrically connected to an inner coil conductor 190 by means of a helix shaft 160 manufactured by an electrically conductive material such as platinum, gold, tantalum, titanium, iridium or an alloy of any of these materials, such as platinum/iridium, e.g. Pt/Ir 90/10 or 80/20. The helix shaft 160 or at least a portion thereof is preferably made of a high density, electrically conductive material to be radiopaque. The proximal end of the helix electrode 110 and the distal end of the inner coil conductor 190 may be attached by, for instance laser welding or the like, to the opposite ends of the helix shaft 160. The shaft 160 is journaled for rotation and axial movement within a sleeve or coupling 170 and includes a radially extending flange defining a proximal, radially-extending surface engageable against a distal extremity of the sleeve to limit the retraction of the helix electrode 110. The sleeve 170 is preferably electrically conductive and secured to an inner conductive tube 150 ending at the collar 120. The sleeve 170 and the conductive tube 150 are preferably made of an electrically conducting metal, such as titanium, or metal alloy, such as MP35N^{®} or stainless steel.

The proximal portion of the sleeve 170 has a counterbore terminating at a distal end wall. An electrically conductive tubular abutment 164, such as of MP35N^{®} or the like, L-shaped in cross section, has an axial portion connected, e.g. welded, to the proximal end of the helix shaft 160 and a flange projecting radially within the counterbore of the sleeve 170. Thus, the abutment 164 being secured to the shaft 160 is movable rotationally and axially with the shaft 160 relative to the sleeve 170.

Contained within the counterbore is an electrically conductive, expandable/contractable contact member, preferably in the form of a metallic compression spring 162 of, for instance, MP35N^{®} or like material. In such a case, electrically continuity is thereby established between the collar 120 and the terminal contact pin of the connector assembly via the inner tube 150, the sleeve 170, the contact spring 162, the L-shaped abutment 164 and the inner conductor coil 190. The contact spring 162 is extended or contracted depending on the extension or retraction of the helix electrode 110.

An outer insulating tube 130, for instance of silicone rubber or polyurethane, a Elast-Eon^{®} polymer (trademark of Aor Tech International, a polymer of silicone with polyurethane), such as Elast-Eon^{®} 2A, 2D, 3A, 3LH or HF, extends between the proximal face of the collar 120 and the distal extremity of a ring electrode member 210. A corresponding insulating tube 130 also covers the main lead body by extending from the proximal extremity of the ring electrode 210 up to the connector assembly.

Projecting radially inwardly from the inner surface of the inner header tube 150 is a post 152 interposed between adjacent turns of the helix electrode 110. In this fashion, rotation of the helix electrode 110 forces the electrode 110 to advance or retract within the lead body header.

The ring electrode 200 of the present invention is in mechanical and electrical contact with a terminal of the collector assembly, such as the collector ring, through an outer coil conductor 192. The two coil conductors 190, 192 are electrically insulated by a longitudinally extending insulating tube 180, such as made of silicone rubber, polyeurethane, Elast-Eon^{®} or the like. This insulator 180 is disposed between the coils 190, 192 to prevent electrical contact between the conductors 190, 192 and between the ring electrode member 210 and the inner conductor coil 190.

The figure schematically illustrates an embodiment of a ring electrode 200 according to the present invention. The ring electrode 200 comprises an electrically conducting electrode member 210 connected to a coil adapter 220 also made of a conducting material. This coil adapter 220 mechanically and electrically connects the electrode member 210 to the outer conductor coil 192.

The ring electrode 200 of the present invention comprises at least two ring elements 210, 220 made of different electrically conducting material. Thus, a first element is the actual electrode member 210 having a portion that faces the outside environment when implanted in a patient body. This electrode member 210 must therefore be made of a biocompatible conducting material having corrosion-resistance and material properties that are required for the rather harsh environment it will face following implantation.

Typical materials for the electrode member 210 include metal or metal alloy materials and preferably electrically conducting metal (alloy) materials. Examples of metal materials include platinum, titanium, tantalum, iridium and niobium, and different alloys thereof, such as titanium alloys or platinum/ iridium (Pt/Ir) alloys, including Pt/Ir 90/10 or Pt/Ir 80/20. Also other metal alloy materials can be used including a nickel-cobalt-chromium-molybdenum alloy, such as MP35N^{®} (trademark of SPS Technologies, Inc.) or 35N LT^{®} (trademark of Fort Wayne Metals Research Products Corp.), or an iron-nickel-cobalt alloy, such as Kovar^{®} (trademark of Carpenter Technology Corp.). A preferred conducting material of the electrode member 210 is titanium.

Traditionally the ring electrode member 210 is mechanically and electrically connected directly to the outer conductor coil 192. However, generally the conductor coil 192 is made of a second conducting material different from the material of the electrode member 210 or at least there is a desire to use a second different material. There may though be problems when connecting the electrode member 210 directly to the coil conductor 192 and where these two elements are made of different materials. For instance, the main connecting technique employed in lead assembly, welding, may cause problems as a weld between, for instance, a titanium electrode member and a MP35N^{®} conductor coil may become brittle and may have problems withstanding the mechanical strain, which arise during implementation and use of the lead.

The present invention solves this problem by introducing a coil adapter 220 to the ring electrode 200. This coil adapter 220 acts like a bridge between the electrode member 210 and the conductor coil 192. The coil adapter 220 is made of a second conducing material, typically the same material as the outer conductor coil 192 or a matching material that allows formation of a reliable and robust electrical and mechanical connection to the conductor coil 192, preferably through welding, such as laser welding: Preferred materials of the coil adapter 220 include conducting metal (alloy) materials, such as platinum, titanium, tantalum, iridium and niobium, and different alloys thereof, such as titanium alloys or platinum/iridium (Pt/Ir) alloys, including Pt/Ir 90/10 or Pt/Ir 80/20. Also other metal alloy materials can be used including a nickel-cobalt-chromium-molybdenum alloy, such as MP35N^{®} (trademark of SPS Technologies, Inc.) or 35N LT^{®} (trademark of Fort Wayne Metals Research Products Corp.), or an iron-nickel-cobalt alloy, such as Kovar^{®} (trademark of Carpenter Technology Corp.). A preferred conducting material of the coil adapter is MP35N^{®} or 35N LT^{®}. The coil adapter 220 is preferably made of the same material as the outer conductor or conductor coil 192, or at least a material that can be efficiently and reliably welded thereto.

The electrode member 210 and the coil adapter 220 of the present invention are mechanically and electrically connected at least partly through spark plasma sintering, which is described further herein. This means that the two ring electrode elements 210, 220 can be directly connected to each other through spark plasma sintering to thereby obtain a robust, strain- and corrosion-resistant mechanical and electrical connection between the coil adapter and the electrode member. Alternatively, spark plasma sintering is used for achieving an indirect connection between the coil adapter 220 and the electrode member 210. In this latter case, one or more additional ring electrode elements can be used for connecting the coil adapter 220 and the electrode member 210. Though, the connection of one such additional element to the coil adapter 220 and/or the electrode member 210 and/ or the interconnection of two such additional elements is achieved through spark plasma sintering.

Thus, connecting the coil adapter 220 of the invention to the electrode member 210 at least partly by spark plasma sintering encompasses a direct mechanical and electrical connection between the elements by spark plasma sintering but also encompasses an indirect mechanical and electrical connection using one or more additional connection elements, where at least one connection between the elements of the ring electrode 200 is obtained through spark plasma sintering, though other such connections may be performed through other techniques, such as welding.

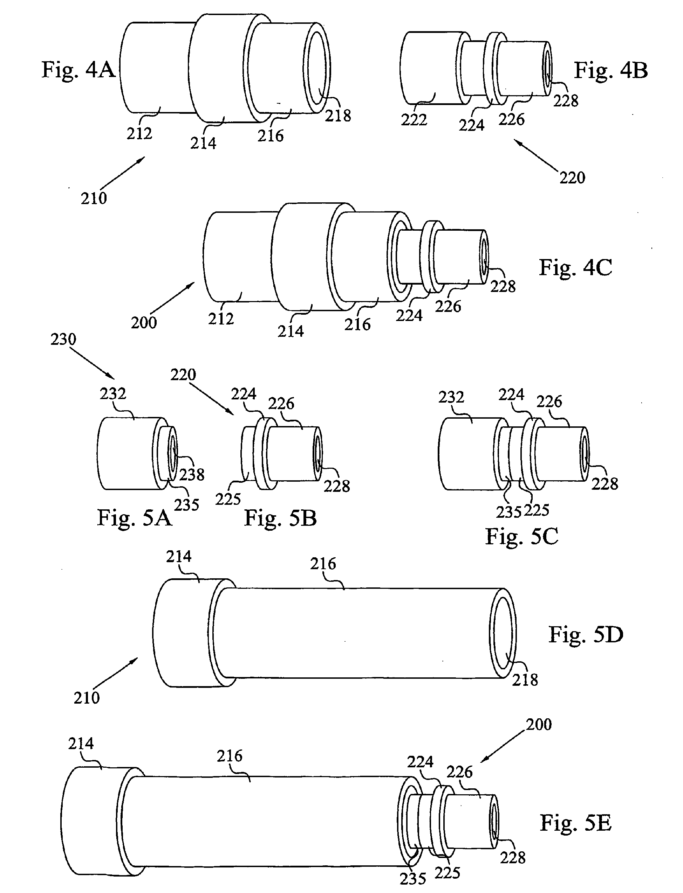

Figs. 4A to 4C illustrate a close-up view of the electrode member 210 and the coil adapter 220 according to an embodiment of the present invention. As is illustrated in Fig. 4A, the electrode member 210 can generally be in the form of a conducting tubular member having a central bore 218 through which the inner coil, insulating tube and possibly a proximal portion of the helix shaft will run. The electrode 210 has a shoulder 214, a lateral surface of which constitutes the outwardly facing electrode surface of the ring electrode 200. Thus, this shoulder surface may be used for stimulating and/or sensing surrounding tissue when implanted at a site in a patient body. The shoulder 214 has a neighboring proximal electrode member portion 216 and a corresponding distal portion 212. The shoulder 214 extends radially beyond the outer circumference of these end portions 212, 216 for allowing insulating tubes to be threaded on the end portions 212, 216 thereby leaving the lateral electrode surface 214 uncovered by any insulating material.

Fig. 4B correspondingly illustrates the coil adapter 220 of the present invention. The coil adapter 220 is generally in the form of a tube having a central bore 228, through which the inner coil conductor, inner insulating tube and possibly proximal end of helix shaft may run after assembling the lead header parts. The distal adapter portion is in the form of a shoulder portion 222 adapted for connection to the proximal portion 216 of the electrode member 210. According to an embodiment of the present invention, the electrode member 210 and the coil adapter 220 are mechanically and electrically connected through spark plasma sintering, which is described further herein.

In a first implementation, the outer diameter of the distal adapter portion 222 matches the outer diameter of the proximal electrode portion 216. In such a case, the ends of the coil adapter 220 and the electrode member 210 are anchored together to form a ring electrode. This means that the circular distal end portion 222 of the coil adapter 220 will be connected by spark plasma sintering to the circular proximal end portion 216 of the electrode member 210.

In a second embodiment, an outer diameter of the distal adapter portion 222 matches the inner diameter of the proximal electrode portion 216. This means that the shoulder portion 222 is adapted for insertion, at least partly, into the bore 218 of the electrode member 210 as illustrated in Fig. 4C. In such a case, the lateral surface of the shoulder 222 or at least a portion of the lateral surface becomes connected through spark plasma sintering to, at least a portion of, an inner surface of the proximal electrode portion 218. In a preferred embodiment, there is a radially inwardly protruding abutment or shoulder in the bore of the electrode member 210 that functions as a stop when introducing the coil adapter 220 into the bore 218. This stop is more clearly illustrated in Fig. 3.

In a third implementation, an outer diameter of the proximal electrode portion 216 matches the inner diameter of the distal adapter portion 222. In this implementation, the proximal electrode portion 216 is inserted into the bore 228 of the coil adapter 220 and connected thereto through spark plasma sintering.

The coil adapter 220 preferably comprises a second outwardly protruding shoulder 224 covering the circumference or a portion of the circumference of the coil adapter 220. This shoulder 224 acts like a stop and possibly anchoring element to the outer conductor coil. Thus, the proximal coil portion is threaded on the proximal adapter portion 226 up to the coil adapter. At this position the conductor coil can be attached to the shoulder 224 or the lateral surface of the proximal adapter portion 226, typically by welding.

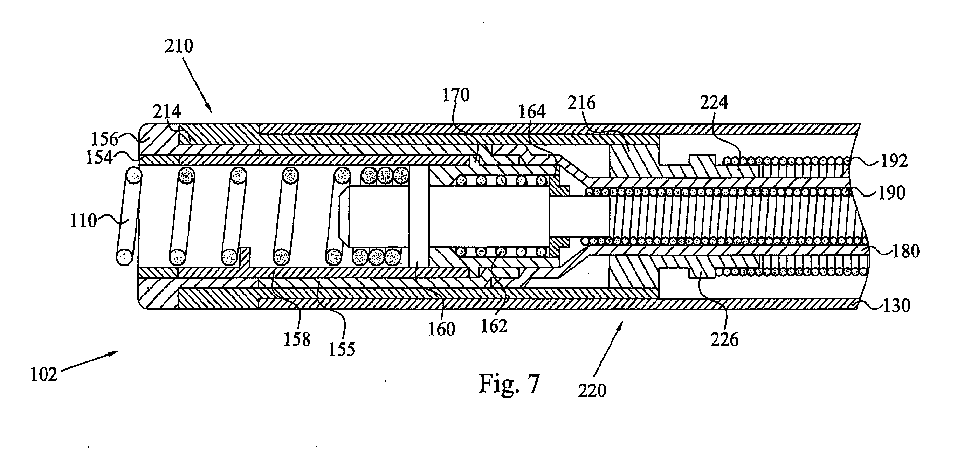

The ring electrode illustrated in Figs. 4A to 4C is adapted for implementation in a lead header as illustrated in Fig. 3. However, the ring electrode of the present invention can alternatively be used in so called far-field signal reduction (FSR) leads having a comparatively shorter tip to ring spacing. In such a leads, the ring electrode member is positioned much closer to the distal lead end than what is illustrated in Fig. 3. The distal end 102 of such a FSR-capable lead is illustrated in Fig. 7. It is evident from the figure that the electrode member 210 has its ring electrode 214 much closer to the tip as compared to Fig. 3. The electrode member 210 has a longitudinally extending tube portion 216 that connects the outwardly facing lateral electrode surface 214 with the coil adaptor 220 and the outer conductor coil 192.

The distal FSR lead portion 102 comprises a header 158, such as made of titanium, extending from the sleeve 170 up towards the distal lead end. The opposite side of the header 158 is preferably connected to a marker ring 154, such as made of Pt/Ir. The marker ring 154 is covered by an insulating header cap 156, which can be made of any insulating material generally employed in medical leads, such as a silicone material, polyurethane, or Elast-Eon^{®}. An insulating tubing 155 is provided around the header 158, extending down to covering at least a portion of the sleeve 170. This can also be made of different insulating materials, such as polytetrafluoroethylene (PTFE). This insulating tubing 155 electrically insulates the electrode member 214 of the ring electrode from the header 158 and the sleeve/coupling 170. The inner insulator 180 preferably extends, in this FSR lead, up to and covers at least an end portion of the insulating tubing 155. The remaining lead header elements are similar to those illustrated in Fig. 3.

Figs. 5A to 5E illustrate a ring electrode assembly according to the present invention adapted for usage in implantable leads adopting the FSR technology. The figures also illustrate usage of a so-called weld adapter 230 as a bridging element between the coil adapter 220 and the electrode member 210 in the ring electrode assembly.

Fig. 5A illustrates an embodiment of the weld adapter 230. In this embodiment, the weld adapter 230 is made of the first conducting material, i.e. the same material as the electrode member 210 or at least a matching material that can be welded to the electrode member 210 to provide a robust and reliable mechanical and electrical connection between the electrode member 210 and the weld adapter 230. The weld adapter 230 is in the form of a tube or cylinder with a central bore 238 through which the inner conductor coil, inner insulating tube and possibly proximal shaft portion extend. A proximal end portion 235 of the weld adapter 230 is in this embodiment adapted for connection to a matching distal end portion 225 of the coil adapter through spark plasma sintering. The resulting sub-assembly is illustrated in Fig. 5C. It is noted in this figure that the sub-assembly preferably has a same general shape as the coil adapter of Fig. 4B. As a consequence, the distal end portion 232 of the weld adapter forms a shoulder having an outer diameter matching an inner diameter of the bore 218 of the electrode member (Fig. 5D). Thus, at least a portion of this shoulder 232 is adapted for insertion into the bore 218 and mechanically and electrically connected thereto by welding the inner tube surface of the proximal electrode portion 216 to the lateral surface of the shoulder 232. The final ring electrode assembly 200 is illustrated in Fig. 5E.

In this embodiment, the weld adapter 230 and coil adapter 220 are interconnected through spark plasma sintering. The weld adapter 230 is in turn welded to electrode member 210 to thereby form a mechanical and electrical connection between the coil adapter 220 and the electrode member 210 formed partly by spark plasma sintering.

It is anticipated by the present invention that in another embodiment, the proximal electrode portion 216 is instead partly inserted into the bore 238 of the weld adapter 230 and welded thereto. Alternatively, the diameter of the proximal electrode portion 216 matches the diameter of the distal weld adapter portion 232. In such a case, the two elements 210, 230 can be welded together end-to-end.

Furthermore, in an alternative embodiment the weld adapter 230 is made of the second conducting material, i.e. the same material as the coil adapter 220 or at least a matching material that can be welded thereto to form a reliable, robust and non-brittle weld therebetween. In such a case, the weld adapter 230 is connected to the electrode member 210 by spark plasma sintering. The resulting sub-assembly can then be connected to the coil adapter 220 by welding the weld adapter 230 to the coil adapter 210.

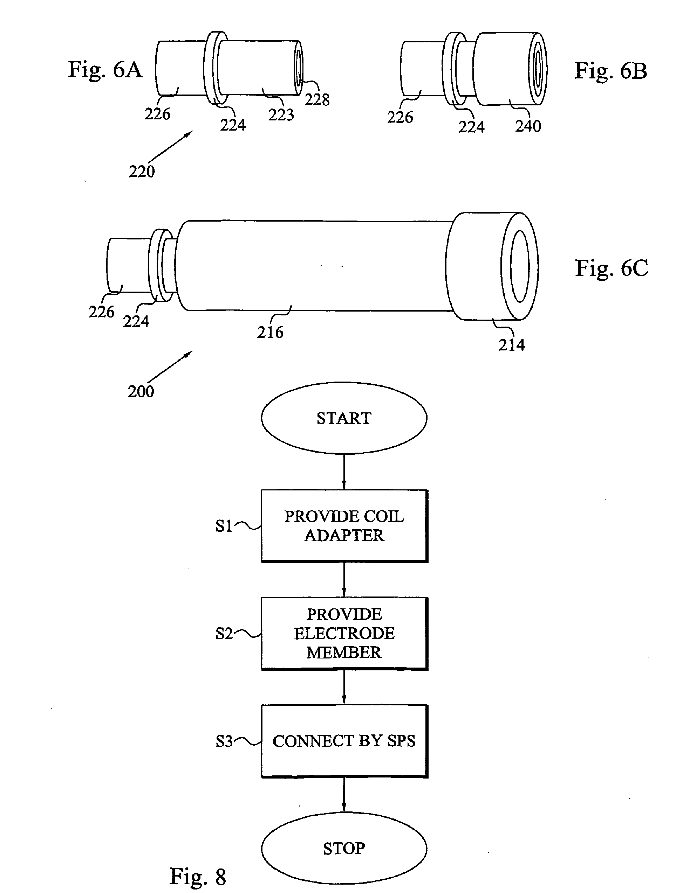

Figs. 6A to 6C illustrate a ring electrode assembly 200 adapted for usage in a FSR lead but having another weld adapter embodiment. Fig. 6A illustrates the coil adapter having proximal 226 and distal 223 adapter portions separated by a shoulder 224 acting as coil stop and/or coil anchoring structure. The weld adapter 240 is in this embodiment shaped as a ring or tubular member threaded on at least a portion of the distal coil adapter portion 223. The two adapters 220, 240 are mechanically and electrically interconnected by spark plasma sintering to thereby concentrically align the weld adapter 240 around a portion 223 of the coil adapter 220. The coil adapter 220 is connected to the electrode member 210 by inserting at least a portion of the weld adapter 240 into the bore 218 at the proximal electrode end and welding the weld adapter 240 to the electrode member 210. The final ring electrode assembly 200 is illustrated in Fig. 6C. In this embodiment, the weld adapter 240 and the electrode member 210 are preferably made of same or at least inter-weldable conducting materials.

In an alternative embodiment, the weld adapter 240 and the coil adapter 220 are made of same or at least inter-weldable conducting materials. The weld adapter is then preferably first spark plasma sintering into the bore 218 of the electrode member 210 or around the proximal end portion of the electrode member 210. In the former case the distal end 223 of coil adapter 210 is introduced into the ring-shaped weld adapter 240 and welded thereto.

In the latter case, the weld adapter 240 is introduced in the bore 228 of the coil adapter 210 and connected by welding.

Fig. 8 is a flow diagram illustrating a method of producing a ring electrode assembly of an implantable lead according to the present invention. The method involves providing an at least partly fabricated coil adapter made of a first conducting material in step S1.

According to the present invention "at least partly fabricated" means a sub-assembly that is either a completely fabricated ring electrode element or at least a partly fabricated raw or start material that can be formed into a fabricated element. As a consequence, at least partly fabricated encompasses providing powder, grain, granule or granulate particles of the first (in the case of coil adapter) or second (in the case of electrode member) conducting material compacted to a shape from which the final shape of the element can be fabricated and formed to a continuous body by spark plasma sintering. At least partly fabricated also encompasses a finally fabricated element that is to be connected, such as by spark plasma sintering, to another element of the ring electrode member. The expression also covers element bodies between these two extremes such as an continuous element body of the conducting material that is connected, such as by spark plasma sintering, to another element of the ring electrode assembly but having a shape different from the final shape of the element. In such a case, the element body can be further processed, such as turned, ground, etched, subjected to electrical discharge machining (EDM), milled, sawed, drawn, tumbled, swaged, forged, welded, following the connection to form the desired final shape and/or surface treatment.

Step S1 can therefore involve, for instance, providing powder particles of the first conducting material and forming, in a die, the particles to a desired shape. Alternatively, a fabricated coil adapter body of the second material, such as illustrated in Figs. 4B, 5B or 6A, is provided in step S1. Furthermore, the step S1 can involve provided a continuous body, such as a cylinder or tubular body, of the first conducting material, where the body has a shape different from the final shape of the coil adapter.

A next step S2 involves providing an at least partly fabricated electrode member made of a second conducting material. This step S2 can, in consistency with step S1, involve providing powder particles of the second material and compacting them to a desired shape. Alternatively, a fabricated electrode member having a shape as illustrated in Figs. 4A, 5D or 6C, is provided in step S2 or alternatively a continuous but not finally processed electrode member body.

A next step S3 involves directly or indirectly connecting the coil adapter and the electrode member at least partly by spark plasma sintering. Furthermore, in the case any of the providing steps S1 and S2 involved providing material particles, the spark plasma sintering operation performed in step S3 also includes forming a continuous body of the material in addition to inter-connecting at least two ring electrode elements.

"Spark plasma sintering" or "SPS" is a sintering technique that applies, in addition to pressure, DC current/voltage pulses directly through a die containing a sample to be formed or samples to be inter-connected. The DC current pulses not only pass through the die by also through the actual sample(s) in the case of conductive samples. As a consequence, heat is generated internally through spark discharge between the particles occurring in the initial stage of the current-voltage pulse. The generation of spark impact pressure, Joule heat and the action of the electric field will result in efficient heating, plastic deformation promotion, high-speed diffusion and material transfer that give an opportunity to conduct low-temperature, short-time sintering of hard-to-sinter materials and bonding of dissimilar materials.

Spark plasma is formed initially of the sintering process and necks between the particles are created. After the initial process, surface, grain-body and volume-diffusion-processes and plastic flow contribute to densification while avoiding coarsening. SPS also facilitates a very high heating or cooling rate (several hundred °K/minute), hence the sintering process is very fast. SPS is also sometimes denoted field assisted sintering technique (FAST) or pulsed electric current sintering (PECS) in the art.

Thus, the expression "spark plasma sintering" as used herein relates to a technique for forming inter-metal bonds between metal (alloy) bodies of different conductive materials through the application of pressure and DC current/voltage pulses through the die and the at least partly fabricated ring electrode elements of the invention.

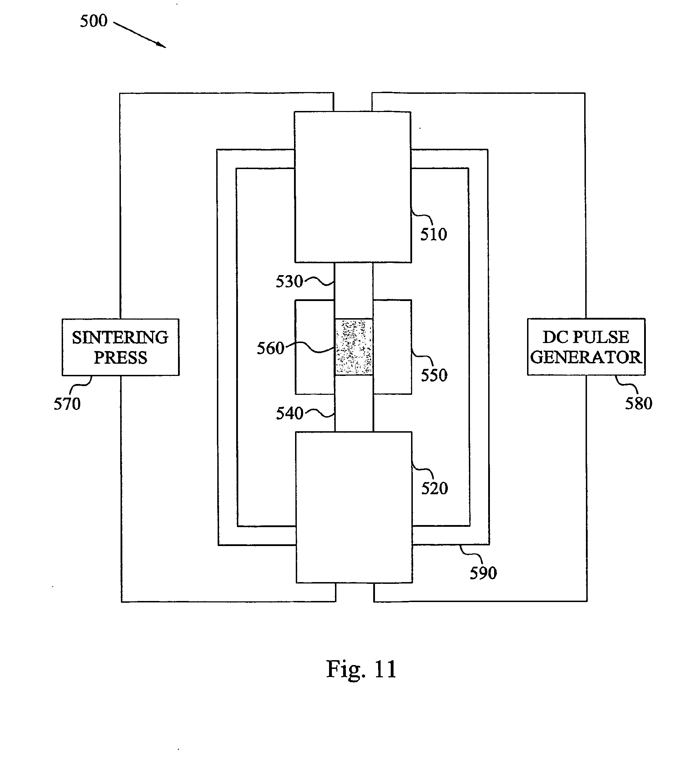

Fig. 11 is a schematic a SPS device 500 that can be used according to the present invention. The device 500 comprises an upper punch electrode 510 connected to an upper punch 530. A corresponding lower punch electrode 520 is connected to a lower punch 540. The two ring electrode elements 560 of the invention to be mechanically and electrically connected by SPS according to the present invention are provided in a sintering (graphite) die 550 between the two punches 530, 540. The sample 560 is enclosed together with the punches 530, 540 and the die 550 into a vacuum chamber 590. A DC pulse generator 580 is connected to the upper 510 and lower 530 punch electrode to thereby apply DC current/voltage pulses over the die 550 and through the electrode elements 560. A sintering press 570 is arranged for controlling an exerted pressure applied by the punches 530, 540 to the electrode elements 560.

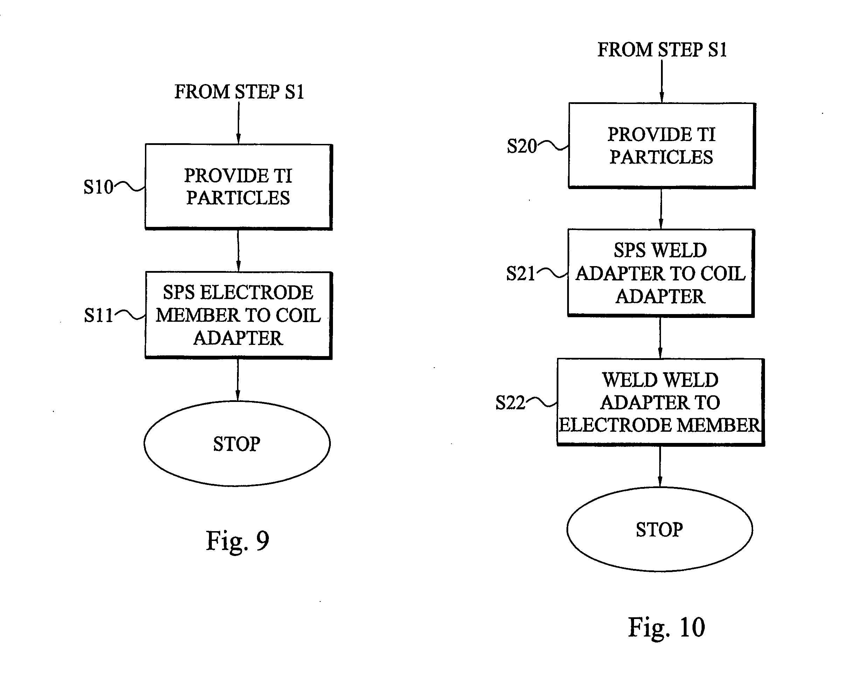

Fig. 9 is a flow diagram illustrating an embodiment of the providing step S2 and connecting step S3 of Fig. 8. The method continues from step S1 of Fig. 9. In a next step S10, powder/grain/granulate/granule particles of the second conducting material, preferably titanium, is provided and formed in the sintering die around the distal portion of the coil adapter and shaped to the final electrode member shape or at least to a cylindrical body. The SPS device is activated to densify the titanium particles into the desired shape and also mechanically connect the resulting electrode member body to the coil adapter to thereby provide the mechanical and electrical connection between these to elements. The method then ends.

The at least fabricated coil adapted can be in the form of powder/grain/granulate/granule particles of the first conducting material, preferably MP35N^{®} or a similar alloy, formed in the die to the final shape of the coil adapter or at least into a body that can be further processed into the final coil adapter shape following the spark plasma sintering. Alternatively, the coil adapter is in the form of a continuous body, either finally fabricated or partly fabricated, inserted into the sintering die together with the titanium particles.

In a further embodiment, the coil adapter and the electrode member are in the form of finally fabricated or partly fabricated continuous bodies of the first and second conducting material, respectively. These two bodies are then inserted into the die and spark plasma sintered together to be directly inter-connected.

Fig. 10 is a flow diagram illustrating another embodiment of the providing step S2 and connecting step S3 of Fig. 8. The method continues from step S1 of Fig. 8. In a next step S20 an at least partly fabricated weld adapter made of the second conducting material is provided in the sintering die. This step S20 can be performed as illustrated in the figure, i.e. by providing titanium particles into the shape of the weld adapter or at least a body which can be further processed into the weld adapter. The weld adapter is then formed and connected to the coil adapter, which is also provided in the die, by spark plasma sintering in step S21. A next step S22 welds the weld adapter to the electrode member to thereby provide an indirect mechanical and electrical connection between the electrode member and the coil adapter.

The above-described discussion of the at least partly fabrication form of the coil adapter and the electrode member also applies to this embodiment, in which the coil adapter and the weld adapter are inter-connected. As was described in the foregoing, in an alternative approach, an at least partly fabricated weld adapter made of the first conducting material is provided in the sintering die together with an at least partly fabricated electrode member made of the second conducting material. The two elements are mechanically inter-connected by spark plasma sintering and then the weld adapter is welded to the coil adapter.

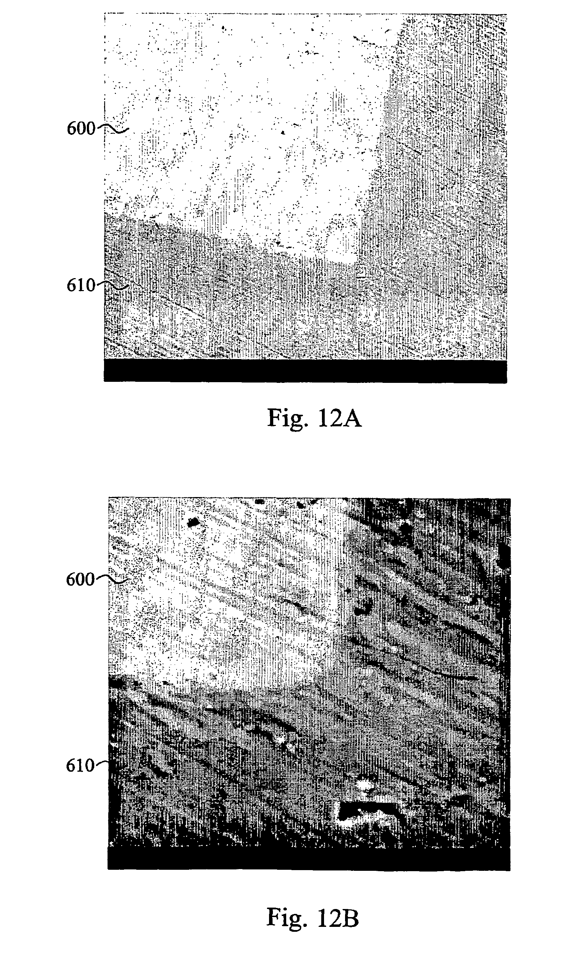

A test body made of a preferred conducting material of the electrode member, i.e. titanium, and a preferred conducting material of the coil adapter, i.e. MP35N^{®}, has been spark plasma sintered at a temperature of 800 °C for 1 minute. The test bodies had a diameter of 12 mm and a height of 3 mm and were sintered from MP35N^{®} and titanium powder. Figs. 12A and 12B are scanning electron microscope images of the joint between the titanium material 600 and the MP35N^{®} material 610 at 500 x magnification (Fig. 12A) and 4000 x magnification (Fig. 12B). As can be seen from the drawings, spark plasma sintering can be used for providing a seamless connection between the two different conducting materials 600, 610 without the formation of any cracks or voids in the joint.

The SPS procedure of the present invention results in a diffusion-based joint between the materials. In a small transition zone around the joint, particles of the materials become inter-mixed to form the diffusion-based, seamless joint.

As is well known in the art, the temperature of the SPS process is dependent on, among others, the particular materials to be sintered and the thickness of the materials or the size of the material particles. Briefly, the sintering temperature should be below the lowest melting point of the materials to the sintered. Furthermore, the smaller the diameter of the material particles, generally the lower sintering temperature can be used. Care must also be taken for materials that are subject to phase transistions so that not an unaccepted, from mechanical and/or electrical properties point of view, is formed in the SPS process. The pressure that is applied during the SPS process is also material dependent and depends on the size of the sintered materials. Generally, the pressure per surface area is rather constant for a given material.

The optimal SPS sintering parameters can be determined by the person skilled in the art through routine tests and table look-ups regarding the particular material combinations. The above described parameter settings can be used as starting points in such an optimization process.

The electrochemical behavior of Ti (grade 2) sintered to MP35N^{®} was investigated. The material combination Ti/MP35N^{®} showed low current density during polarization. The current density is related to the corrosion rate, implying that the investigate material combination joint had low corrosion rate. A reason for this low current density may be attributed to the presence of protective oxide films. The protective oxide film of MP35N^{®} consists of Cr₂O₃. The titanium has a passive surface film in the form of TiO₂. This passive surface film contributes significantly to the corrosion resistance of the materials. The tested material combination showed good corrosion resistance in artificial physiological solutions.

An electrochemical investigation was also tested for another possible metal combination according to the invention, i.e. a PtIr alloy and titanium. The metal combination showed low current density during polarization. The reason for this low current density is the protective oxide (TiO₂) film and the inert behavior of PtIr. Potentiodynamic polarization does not affect Ti and PtIr at all or only to a minor degree. The material combination exhibited good corrosion resistance.

It will be understood by a person skilled in the art that various modifications and changes may be made to the present invention without departure from the scope thereof, which is defined by the appended claims.

## Claims

1. A method of producing a ring electrode (200) of an implantable lead (100), said method comprising:
- providing an at least partly fabricated coil adapter (220) made of a first conducting material; and
- providing an at least partly fabricated electrode member (210) made of a second conducting material, **characterized by:**
- directly or indirectly connecting said coil adapter (220) and said electrode member (210) at least partly by spark plasma sintering.

2. The method according to claim 1, **characterized in that** said providing steps collectively comprise the steps of:
- providing first powder particles of said first conducting material;
- forming said first powder particles into a first predefined shape;
- providing second powder particles of said second conducting material; and
- forming said second powder particles into a second predefined shape.

3. The method according to claim 1 or 2, **characterized in that** said directly or indirectly connecting step comprises spark plasma sintering said at least partly fabricated coil adapter (220) and said at least partly fabricated electrode member (210) to form said electrode member (210) mechanically and electrically connected to said coil adapter (220).

4. The method according to claim 1 or 2, **characterized in that** said directly or indirectly connecting step comprises the steps of:
- providing an at least partly fabricated weld adapter (230; 240) of said second conducting material;
- mechanically and electrically connecting said weld adapter (230; 240) to said coil adapter (220) by spark plasma sintering; and
- welding said weld adapter (230; 240) to said electrode member (210).

5. The method according to claim 4, **characterized in that** said mechanically and electrically connecting step comprises spark plasma sintering said at least partly fabricated weld adapter (230) in the form of a cylinder (232) having an end portion (235) mechanically and electrically connected to said coil adapter (220).

6. The method according to claim 5, **characterized in that** said welding step comprises the steps of:
- introducing at least a portion of a lateral surface of said cylinder (232) into a bore (218) of said electrode member (210); and
- welding said at least a portion of said lateral surface to said electrode member (210).

7. The method according to claim 4, **characterized in that** said mechanically and electrically connecting step comprises spark plasma sintering said at least partly fabricated weld adapter (240) in the form of a tubular member mechanically and electrically connected around said coil adapter (220), whereby said weld adapter (240) being concentrically aligned with said coil adapter (220).

8. The method according to claim 7, **characterized in that** said welding step comprises the steps of:
- introducing at least a portion said weld adapter (240) into a bore (218) of said electrode member (210); and
- welding said at least a portion of said weld adapter (240) to said electrode member (210).

9. A ring electrode (200) of an implantable lead (100) comprising a coil adapter (220) made of a first conducting material and an electrode member (210) made of a second conducting material, **characterized in that** said coil adapter (220) and said electrode member (210) are directly or indirectly connected at least partly by spark plasma sintering to form a diffusion based inter-material joint between said first conducting material and said second conducting material.

10. The ring electrode according to claim 9, **characterized in that** said electrode member (210) is spark plasma sintered to said coil adapter (220).

11. The ring electrode according to claim 9, **characterized by** a weld adapter (230, 240) made of said second conducing material and spark plasma sintered to said coil adapter (220), said electrode member (210) being welded to said weld adapter (230, 240).

12. The ring electrode according to claim 11, **characterized in that** said weld adapter (230) is in the form of a cylinder (232) having an end portion (235) connected by spark plasma sintering to said coil adapter (220).

13. The ring electrode according to claim 12, **characterized in that** at least a portion of a lateral surface of said cylinder (232) is introduced in a bore (218) of said electrode member (210) and welded to said electrode member (210).

14. The ring electrode according to claim 11, **characterized in that** said weld adapter (240) is in the form of a tubular member connected by spark plasma sintering around said coil adapter (220) so that said weld adapter (240) is concentrically aligned with said coil adapter (220).

15. The ring electrode according to claim 14, **characterized in that** at least a portion of said weld adapter (240) is introduced in a bore (218) of said electrode member (210) and welded to said electrode member (210).

16. The ring electrode according to claim 9 to 15, **characterized in that** said first conducting material is a conducting metal alloy and said second conducting material is titanium or titanium alloy.

17. The ring electrode according to claim 16, **characterized in that** said conducting metal alloy is a nickel-cobalt-chromium-molybdenum alloy.

## Patentansprüche

1. Verfahren zur Herstellung einer Ringelektrode (200) einer implantierbaren Leitung (100), wobei das Verfahren Folgendes umfasst:
- Bereitstellen von einem zumindest teilgefertigten Wendeladapter (220), der aus einem ersten leitenden Material gefertigt wird; und
- Bereitstellen von einem zumindest teilgefertigten Elektrodenelement (210), das aus einem zweiten leitenden Material gefertigt wird; **gekennzeichnet durch:**
- direktes oder indirektes Verbinden des Wendeladapters (220) und des Elektrodenelements (210) zumindest teilweise **durch** Spark-Plasma-Sintern.

2. Verfahren nach Anspruch 1, **dadurch gekennzeichnet, dass** die Bereitstellungsschritte gemeinsam folgende Schritte umfassen:
- Bereitstellen erster Pulverpartikel aus dem ersten leitenden Material;
- Formen der ersten Pulverpartikel zu einer ersten vordefinierten Form;
- Bereitstellen zweiter Pulverpartikel aus dem zweiten leitenden Material; und
- Formen der zweiten Pulverpartikel zu einer zweiten vordefinierten Form.

3. Verfahren nach Anspruch 1 oder 2, **dadurch gekennzeichnet, dass** der Schritt des direkten oder indirekten Verbindens das Spark-Plasma-Sintern des zumindest teilgefertigten Wendeladapters (220) und des zumindest teilgefertigten Elektrodenelements (210) umfasst, um das Elektrodenelement (210) zu formen, das mechanisch und elektrisch mit dem Wendeladapter (220) verbunden ist.

4. Verfahren nach Anspruch 1 oder 2, **dadurch gekennzeichnet, dass** der Schritt des direkten oder indirekten Verbindens folgende Schritte umfasst:
- Bereitstellen von einem zumindest teilgefertigten Schweißadapter (230; 240) aus dem zweiten leitenden Material;
- mechanisches und elektrisches Verbinden des Schweißadapters (230; 240) mit dem Wendeladapter (220) mittels Spark-Plasma-Sintern; und
- Verschweißen des Schweißadapters (230; 240) mit dem Elektrodenelement (210).

5. Verfahren nach Anspruch 4, **dadurch gekennzeichnet, dass** der Schritt des mechanischen und elektrischen Verbindens das Spark-Plasma-Sintern von dem zumindest teilgefertigten Schweißadapter (230) in Form eines Zylinders (232) umfasst, der einen Endabschnitt (235) aufweist, der mechanisch und elektrisch mit dem Wendeladapter (220) verbunden wird.

6. Verfahren nach Anspruch 5, **dadurch gekennzeichnet, dass** der Schweißschritt folgende Schritte umfasst:
- Einführen von zumindest einem Abschnitt einer Seitenfläche des Zylinders (232) in eine Bohrung (218) des Elektrodenelements (210); und
- Verschweißen des mindestens einen Abschnitts der Seitenfläche mit dem Elektrodenelement (210).

7. Verfahren nach Anspruch 4, **dadurch gekennzeichnet, dass** der Schritt des mechanischen und elektrischen Verbindens das Spark-Plasma-Sintern von dem zumindest teilgefertigten Schweißadapter (240) in Form eines röhrenförmigen Elements umfasst, das mechanisch und elektrisch um den Wendeladapter (220) herum verbunden wird, wodurch der Schweißadapter (240) konzentrisch nach dem Wendeladapter (220) ausgerichtet wird.

8. Verfahren nach Anspruch 7, **dadurch gekennzeichnet, dass** der Schweißschritt folgende Schritte umfasst:
- Einführen von zumindest einem Abschnitt des Schweißadapters (240) in eine Bohrung (218) des Elektrodenelements (210); und
- Verschweißen des zumindest einen Abschnitts des Schweißadapters (240) mit dem Elektrodenelement (210).

9. Ringelektrode (200) einer implantierbaren Leitung (100), umfassend einen Wendeladapter (220), der aus einem ersten leitenden Material gefertigt ist, und ein Elektrodenelement (210), das aus einem zweiten leitenden Material gefertigt ist, **dadurch gekennzeichnet, dass** der Wendeladapter (220) und das Elektrodenelement (210) direkt oder indirekt zumindest teilweise mittels Spark-Plasma-Sintern verbunden sind, um zwischen dem ersten leitenden Material und dem zweiten leitenden Material eine Zwischenmaterialverbindung auf der Grundlage von Diffusion herzustellen.

10. Ringelektrode nach Anspruch 9, **dadurch gekennzeichnet, dass** das Elektrodenelement (210) mit dem Wendeladapter (220) mittels Spark-Plasma-Sintern verbunden ist.

11. Ringelektrode nach Anspruch 9, **gekennzeichnet durch** einen Schweißadapter (230, 240), der aus dem zweiten leitenden Material gefertigt und mit dem Wendeladapter (220) mittels Spark-Plasma-Sintern verbunden ist, wobei das Elektrodenelement (210) mit dem Schweißadapter (230, 240) verschweißt ist.

12. Ringelektrode nach Anspruch 11, **dadurch gekennzeichnet, dass** der Schweißadapter (230) in Form eines Zylinders (232) mit einem Endabschnitt (235) vorliegt, der mittels Spark-Plasma-Sintern mit dem Wendeladapter (220) verbunden ist.

13. Ringelektrode nach Anspruch 12, **dadurch gekennzeichnet, dass** zumindest ein Abschnitt einer Seitenfläche des Zylinders (232) in eine Bohrung (218) des Elektrodenelements (210) eingeführt und mit dem Elektrodenelement (210) verschweißt ist.

14. Ringelektrode nach Anspruch 11, **dadurch gekennzeichnet, dass** der Schweißadapter (240) in Form eines röhrenförmigen Elements vorliegt, das mittels Spark-Plasma-Sintern um den Wendeladapter (220) herum verbunden ist, sodass der Schweißadapter (240) konzenfrisch nach dem Wendeladapter (220) ausgerichtet ist.

15. Ringelektrode nach Anspruch 14, **dadurch gekennzeichnet, dass** zumindest ein Abschnitt des Schweißadapters (240) in eine Bohrung (218) des Elektrodenelements (210) eingeführt und mit dem Elektrodenelement (210) verschweißt ist.

16. Ringelektrode nach Anspruch 9 bis 15, **dadurch gekennzeichnet, dass** es sich bei dem ersten leitenden Material um eine leitende Metalllegierung handelt und es sich bei dem zweiten leitenden Material um Titan oder eine Titanlegierung handelt.

17. Ringelektrode nach Anspruch 16, **dadurch gekennzeichnet, dass** es sich bei der leitenden Metalllegierung um eine Nickel-Cobalt-Chrom-MolybdänLegierung handelt.

## Revendications

1. Procédé de production d'une électrode annulaire (200) pour sonde implantable (100), ledit procédé comprenant :
- mettre à disposition un adaptateur de bobine (220) au moins partiellement fini constitué d'un premier matériau conducteur ; et
- mettre à disposition un élément d'électrode (210) au moins partiellement fini constitué d'un second matériau conducteur, **caractérisé par :**
- la connexion directe ou indirecte dudit adaptateur de bobine (220) et dudit élément d'électrode (210) au moins partiellement par frittage flash (spark plasma sintering).

2. Procédé selon la revendication 1, **caractérisé en ce que** lesdites étapes de mise à disposition comprennent collectivement les étapes consistant à :
- mettre à disposition des premières particules de poudre dudit premier matériau conducteur ;
- former lesdites premières particules de poudre en une première forme prédéfinie ;
- mettre à disposition des secondes particules de poudre dudit second matériau conducteur ; et
- former lesdites secondes particules de poudre en une seconde forme prédéfinie.

3. Procédé selon la revendication 1 ou 2, **caractérisé en ce que** ladite étape de connexion directe ou indirecte comprend le frittage flash dudit adaptateur de bobine (220) au moins partiellement fini et dudit élément d'électrode (210) au moins partiellement fini pour former ledit élément d'électrode (210) connecté mécaniquement et électriquement audit adaptateur de bobine (220).

4. Procédé selon la revendication 1 ou 2, **caractérisé en ce que** ladite étape de connexion directe ou indirecte comprend les étapes consistant à :
- mettre à disposition un adaptateur de soudage (230 ; 240) au moins partiellement fini en ledit second matériau conducteur ;
- connecter mécaniquement et électriquement ledit adaptateur de soudage (230 ; 240) audit adaptateur de bobine (220) par frittage flash ; et
- souder ledit adaptateur de soudage (230 ; 240) audit élément d'électrode (210).

5. Procédé selon la revendication 4, **caractérisé en ce que** ladite étape de connexion mécanique et électrique comprend le frittage flash dudit adaptateur de soudage (230) au moins partiellement fini sous forme d'un cylindre (232) présentant une partie d'extrémité (235) connectée mécaniquement et électriquement audit adaptateur de bobine (220).

6. Procédé selon la revendication 5, **caractérisé en ce que** ladite étape de soudage comprend les étapes consistant à :
- introduire au moins une partie d'une surface latérale dudit cylindre (232) dans un alésage (218) dudit élément d'électrode (210) ; et
- souder ladite au moins une partie de ladite surface latérale audit élément d'électrode (210).

7. Procédé selon la revendication 4, **caractérisé en ce que** ladite étape de connexion mécanique et électrique comprend le frittage flash dudit adaptateur de soudage (240) au moins partiellement fini sous forme d'un élément tubulaire connecté mécaniquement et électriquement autour dudit adaptateur de bobine (220), ce par quoi ledit adaptateur de soudage (240) est aligné de manière concentrique avec ledit adaptateur de bobine (220).

8. Procédé selon la revendication 7, **caractérisé en ce que** ladite étape de soudage comprend les étapes consistant à :
- introduire au moins une partie dudit adaptateur de soudage (240) dans un alésage (218) dudit élément d'électrode (210) ; et
- souder ladite au moins une partie dudit adaptateur de soudage (240) audit élément d'électrode (210).

9. Électrode annulaire (200) pour sonde implantable (100) comprenant un adaptateur de bobine (220) constitué d'un premier matériau conducteur et un élément d'électrode (210) constitué d'un second matériau conducteur, **caractérisée en ce que** ledit adaptateur de bobine (220) et ledit élément d'électrode (210) sont connectés directement ou indirectement au moins partiellement par frittage flash pour former un assemblage entre matériaux entre ledit premier matériau conducteur et ledit second matériau conducteur reposant sur la diffusion.

10. Électrode annulaire selon la revendication 9, **caractérisée en ce que** ledit élément d'électrode (210) est relié par frittage flash audit adaptateur de bobine (220).

11. Électrode annulaire selon la revendication 9, **caractérisée par** un adaptateur de soudage (230, 240) constitué dudit second matériau conducteur et relié par frittage flash audit adaptateur de bobine (220), ledit élément d'électrode (210) étant soudé audit adaptateur de soudage (230, 240).

12. Électrode annulaire selon la revendication 11, **caractérisée en ce que** ledit adaptateur de soudage (230) est sous forme d'un cylindre (232) présentant une partie d'extrémité (235) connectée par frittage flash audit adaptateur de bobine (220).

13. Électrode annulaire selon la revendication 12, **caractérisée en ce qu**'au moins une partie d'une surface latérale dudit cylindre (232) est introduite dans un alésage (218) dudit élément d'électrode (210) et soudée audit élément d'électrode (210).

14. Électrode annulaire selon la revendication 11, **caractérisée en ce que** ledit adaptateur de soudage (240) est sous forme d'un élément tubulaire connecté par frittage flash autour dudit adaptateur de bobine (220) de sorte que ledit adaptateur de soudage (240) est aligné de manière concentrique avec ledit adaptateur de bobine (220).

15. Électrode annulaire selon la revendication 14, **caractérisée en ce qu**'au moins une partie dudit adaptateur de soudage (240) est introduite dans un alésage (218) dudit élément d'électrode (210) et soudée audit élément d'électrode (210).

16. Électrode annulaire selon les revendications 9 à 15, **caractérisée en ce que** ledit premier matériau conducteur est un alliage métallique conducteur et ledit second matériau conducteur est du titane ou un alliage de titane.

17. Électrode annulaire selon la revendication 16, **caractérisée en ce que** ledit alliage métallique conducteur est un alliage nickel-cobalt-chrome-molybdène.
